# EUROPEAN PATENT SPECIFICATION

(11) **EP 1 867 290 B1**
(45) Date of publication and mention of the grant of the patent: **27.02.2013**
(21) Application number: 07252105.7
(22) Date of filing: 22.05.2007
(51) Int. Cl.: A61B 17/22, A61F 2/01

(54) **Vascular thrombectomby apparatus and method of use**
Thrombektomievorrichtung und Anwendungsverfahren
Appareil de trombectomie vasculaire et procédé d'utilisation

(30) Priority: 13.06.2006 US 452038
(43) Date of publication of application: 19.12.2007
(73) Proprietor: Cordis Corporation, Miami Lakes, FL 33014 (US)
(72) Inventor: Tanaka, Don A., Saratoga, CA 95070 (US); Souza, Alison M., Santa Clara, CA 95050 (US)
(74) Representative: Belcher, Simon James

(56) References cited:
- EP-A- 1 346 703
- WO-A-01/34041
- WO-A-98/38920
- WO-A-03/015840
- WO-A-2004/062513
- WO-A-2004/093696
- WO-A-2004/093966

## Description

This invention generally relates to an apparatus used to filter or remove matter from within a body conduit. In particular, this invention relates to a self-expanding device used in interventional procedures such as thrombectomy or embolectomy that resists plastic deformation as it engages, filters and/or removes matter that is entrapped in a conduit of a body.

Interventional procedures are often necessary to restore the flow of fluids in conduits of the human body. For example, percutaneous interventional procedures may be employed to introduce a stent into the vasculature of a human body to restore the proper flow of blood. During this process matter such as emboli or thrombi may be introduced into the blood stream. In addition, matter may be naturally present in the blood stream or in a conduit of a human body. It is necessary to filter or remove the matter from the conduit to avoid adverse physical affects such as ischemic stroke.

A typical interventional procedure involves introducing a guidewire into the vasculature of the patient. The guidewire is routed and advanced beyond the point in the conduit where the matter to be removed resides. The guidewire serves as a track over which a catheter or other interventional device can be advanced. Once the catheter is in place, a variety of devices can be advanced through the catheter and used to capture, filter and/or remove the matter. For example, filters of various types have found use in trapping blood clots and other debris released into the bloodstream. A typical filter comprises a frame with a basket mounted thereon such that an opening is formed at the proximal end thereof. Once the interventional procedure, such as placement of a stent or balloon angioplasty, is complete or the filter is placed in proximity to a thrombi or emboli, the filter is pulled in a desired direction closing the basket and entrapping the matter.

Many filters are only partially effective in capturing debris resulting from intervention procedures or naturally occurring debris lodged in the vasculature because deployment of the filter within the conduit may not provide complete filtration. This may result from failing to maintain an optimum fit of the filter within the vessel or conduit wall resulting in a gap there between. Where a filter basket is employed another drawback may be encountered if the basket does not fully deploy within the vessel.

Existing filter devices also fail to exhibit the necessary characteristics to capture emboli or thrombi that are attached to a vessel wall or lodged within a vessel. For example, thrombi are often fixedly attached or lodged within a vessel and significant force is required to dislodge them. If a filter does not have the proper modulus of elasticity, it will deform and fail to capture or remove the embolic material. An obvious solution to this challenge is to increase the rigidity of the filter. This approach, however, is not well suited for small conduits such as the vasculature located within the human brain, a location where ischemic stroke, blockage of blood vessels by thrombi, originates. In order to fit within small vessels the filter must have commensurate dimensions while also exhibiting the proper modulus of elasticity to ensure proper deployment and removal of the matter from the vasculature. This is a difficult challenge since increased stiffness will ensure removal of the thrombi, but prevent proper delivery and deployment of the filter device from a small deployment sheath or catheter into which the device must be folded.

Numerous approaches have been attempted to meet this challenge. US-6,740,061 -Oslund describes a filter device having a frame with a basket attached thereto and a self expanding radial loop for positioning the basket upon deployment. The spacer or loop is positioned such that it is substantially axially aligned with the mouth of the filter basket. The loop urges the guidewire against the inner wall of a vessel ensuring that the basket is properly positioned. In another embodiment described in Oslund, the mouth of the filter basket is defined by the loop. The filter device of Oslund is not contemplated for use in small vessels. Although the loop provides for proper positioning, it interferes with deployment of the filter device in small vasculature as it increases the stiffness of the device when it is compacted to fit within a delivery sheath or catheter.

US-6,589,263 -Hopkins describes a filtration device having a support hoop with a blood permeable sack affixed thereto. In order to allow the filtration device to be delivered without experiencing kinking or increasing the stiffness, the support hoop includes a reduced thickness articulation region. This region permits the filter frame to compactly fold and deploy within small vasculature without kinking or increasing the stiffness of the filter. Although allowing ready deployment, the reduced thickness region inhibits the ability of the filter device to avoid deformation under loads. For example, instead of capturing a clot, which is firmly attached to the wall of a vessel, the filter device of Hopkins may fold due to the increased strain experienced at the articulation region in a manner similar to the device being pulled back into its delivery sheath or catheter.

US-6,203,561 -Ramee describes a filtration device similar to Hopkins. In order to overcome the shortcomings of Hopkins, Ramee provides a first thrombectomy support hoop and a second filter support hoop. As with Hopkins, each of the support hoops has a blood permeable sack attached thereto and contains a reduced thickness articulation region. According to Ramee, substantially all thrombi is captured by the first support hoop and the second hoop acts as a filter. More likely, however, is that as each of the support hoops contacts the thrombi, they will deform due to the increased strain experienced at the articulation region. Even more alarming is that in failing to completely dislodge and capture the thrombi, pieces of the thrombi may be dislodged and travel downstream causing adverse complications.

WO-A-2004/093696 discusses an embolectomy device including a filter basket and a support frame. The support frame has a proximal hoop and one or more rail members. The support frame and filter basket may be coupled to a wire. In some embodiments filter struts vary in thickness to import a desired flexibility characteristic to the device.

Currently, there is no apparatus that can filter or remove matter from the conduit of a human body. In particular, there is no apparatus that can to fit within small conduits and deploy therein while also exhibiting a modulus of elasticity sufficient to ensure removal of matter from the conduit.

According to the invention, an apparatus is provided for removing matter from within a conduit as defined in appended claim 1. The apparatus generally comprises a separation edge which may be attached to a wire at its proximal end, a frame attached to the distal end of the separation edge, and a membrane, which may also be attached to the wire, and disposed over the frame enclosing its interior. The membrane may comprise a net constructed from a vaporized metal deposited on a mandrel. Alternatively, the membrane may comprise a braided tube constructed from wire.

The apparatus may be employed as a filter or as a means for actively dislodging matter from the wall of a conduit. When employed as a filter, the apparatus is positioned downstream of the matter where it ensures that matter does not escape downstream as it is being removed. When employed to actively remover matter from a conduit, the apparatus is positioned downstream of the matter. The apparatus is then pulled proximally whereby it engages the matter and dislodges it from the wall of the conduit.

The frame, membrane and separation edge may be constructed from a super-elastic material. One example of such super elastic material is Nitinol (Ni-Ti). Use of super elastic materials allow for deformation and restraint in a first deformed condition of the apparatus to facilitate deployment within a conduit. For example, the super elastic characteristics allow the apparatus to have a first, contracted shape when mounted within a sheath or other delivery device employed to position the filter within a conduit. The sheath can be steerable, introduced through a guiding catheter, or navigated over a wire through a conduit to a point downstream of the obstruction to be removed or the matter to be filtered. The filter/retrieval apparatus is deployed from the sheath whereby it expands to a second, expanded shape.

The separation edge comprises at least two members joined together at their distal and proximal ends. In one embodiment, the proximal ends of the members may be joined directly to a wire instead of to each other. The separation edge is joined to a wire that is parallel to the longitudinal axis of the apparatus and articulates it for deployment and capture of matter from within the conduit. The members are slanted from the longitudinal axis of the apparatus to provide an angled cutting surface. Upon deployment, the members contact the inside of the conduit forming a tight seal. In one embodiment of the invention, the members are oval shaped to accommodate circular conduits. An opening is defined between the members serving as an inlet through which matter passes after it is dislodged from the walls of the conduit.

The separation edge experiences high stress and strain due to the force required for removing matter that is entrapped within, or fixed to, the walls of the conduit. This can lead to the separation edge deforming as it contacts the matter such that it assumes its first, contracted shape and fails to remove the matter from the conduit. One solution is to increase the modulus of elasticity of the separation edge. Increasing the modulus of elasticity, however, creates difficulty for delivery of the apparatus, especially in small conduits, and complicates deployment.

A frame, attached to the separation edge, allows the separation edge to maintain flexibility by having a lower modulus of elasticity while preventing the separating edge from buckling as it engages matter and removes it from the wall of the conduit. The frame comprises a plurality of struts that are attached to the separation members. A first group of struts may be connected to the separation edge and to a second group of struts that may be joined at the distal end of the frame. The second group of struts may comprise a plurality of outer struts and a plurality of inner struts attached to and interspersed between the outer struts. The frame may be structured so that the stress experienced by the separation edge is evenly distributed across the frame. The frame may take on a variety of spatial configurations such as a truss or a scaffold.

When the apparatus is delivered to a targeted site in the conduit via a sheath or other delivery device it may be contracted within the sheath to a first diameter. Contracting the frame and separation edge to a small diameter increases stiffness thereby limiting the minimum delivery profile achievable. In addition, the apparatus may deform when contracted jeopardizing optimal deployment. If the apparatus fails to properly deploy the separation edge will not assume the proper cutting angle and will fail to seal against the walls of the conduit allowing matter to escape downstream. In order to ensure proper deployment of the apparatus, at least one deployment section is disposed along the separation edge. The deployment section allows the apparatus to assume its first contracted shape, without increasing stiffness, and allows for deployment in a predictable fashion.

In one embodiment of the invention, the deployment section comprises a preformed point deformation such as a kink. The prior art discusses kinks as being detrimental to contraction and deployment of the apparatus. In contrast to the prior art, the present invention employs a kink specifically formed in an area that will assure that the apparatus is folded within the sheath and deployed in a predictable manner.

Embodiments of the invention will now be described by way of example with reference to the accompanying drawings, in which:
Figure 1 is a perspective view of showing the separation edge and frame of the apparatus of the present invention;
Figure 1A is a detailed view of the deployment region shown in region 1A of Figure 1;
Figure 2 is a bottom view of the apparatus of the present invention;
Figure 3 is an assembly view of the apparatus of the present invention; and
Figure 4 is a perspective view showing the apparatus of the present invention deployed from a delivery device;
Figure 5 is a side, cutaway view showing a catheter deployed within a conduit distally of matter to be removed there from;
Figure 6 is a side, cutaway view showing the apparatus of the present invention is deployed within a conduit distally of matter to be removed there from; and
Figure 7 is a side, cutaway view showing the apparatus of the present invention fully deployed within a conduit capturing the matter to be removed there from.

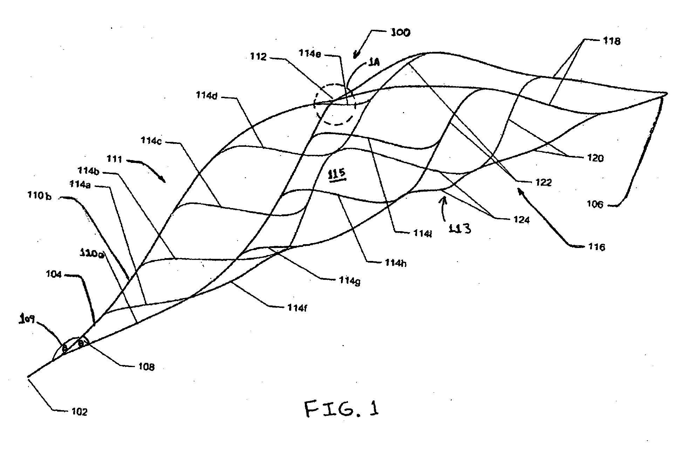

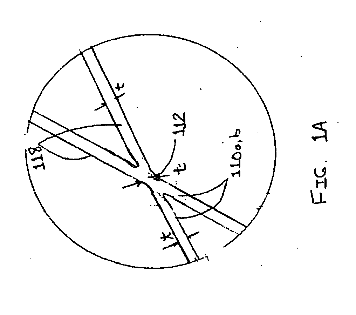

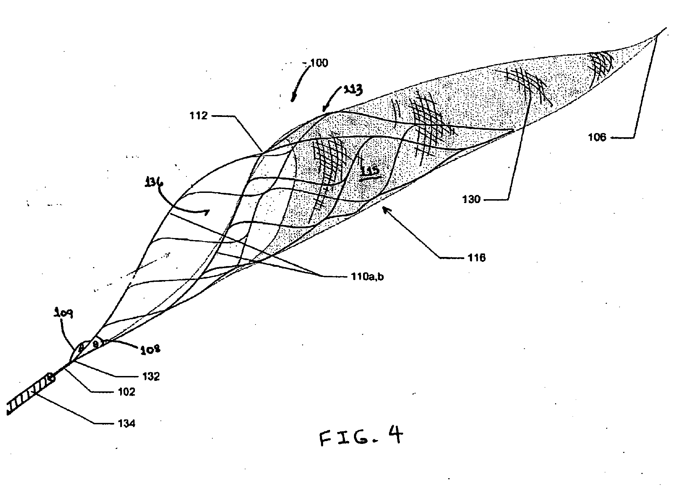

An apparatus for filtering and removing matter from the interior of a conduit will be described with reference to Figures 1-7. As shown in Figure 1 the apparatus 100 of the present invention generally comprises a separation edge 111 attached to a wire or tether 102, a frame 113 attached to the separation edge 111, and, as shown in Figure 4, a membrane or net 130 disposed over the frame 113 enclosing its interior 115.

As shown in Figure 4, the membrane 130 includes openings along its length that allows fluids found within the conduit to pass through the interior 115 of the frame 113, but prevents matter 144 from escaping. For example, in one embodiment of the invention membrane 130 is a blood permeable sac. If desired, the membrane 130 may be attached to the separation edge 111 to provide structural support thereto augmenting the support provided by the frame 113. The membrane 130 may be constructed from a braided tube of material, wire, or a thin metallic film. The metallic film can be set to a specific flat or three-dimensional shape of cylindrical, non-cylindrical, or flat cross section. The creation of the film can be achieved with a wide variety of techniques such as Pulsed Laser Ablation, Physical Vapor Deposition (PVD) including magnetron sputtering, Chemical Vapor Deposition (CVD), Molecular Beam Epitaxy (MBE), thermal deposition (via electron beam or resistive heating), electroplating, dip coating, spin coating or other methods of depositing a metal. The thin films could also be etched via chemical, laser or dry etch methods.

The frame 113, membrane 130 and separation edge 111 are preferably constructed from a super-elastic material. One example of such super elastic material is Nitinol (Ni-Ti). Ni-Ti is utilized in a wide variety of medical applications due to its biomechanical compatibility, its biocompatibility, its fatigue resistance, its uniform plastic deformation, its magnetic resonance imaging compatibility, its ability to exert constant and gentle outward pressure, its dynamic interference, its thermal deployment capability, its elastic deployment capability, its hysteresis characteristics, and is moderately radiopaque.

Nitinol exhibits shape memory and/or super-elastic characteristics. Shape memory characteristics may be simplistically described as follows. A metallic structure, for example, a Nitinol tube that is in an Austenitic phase may be cooled to a temperature such that it is in the Martensitic phase. Once in the Martensitic phase, the Nitinol tube may be deformed into a particular configuration or shape by the application of stress. As long as the Nitinol tube is maintained in the Martensitic phase, the Nitinol tube will remain in its deformed shape. If the Nitinol tube is heated to a temperature sufficient to cause the Nitinol tube to reach the Austenitic phase, the Nitinol tube will return to its original or programmed shape. The original shape is programmed to be a particular shape by well-known techniques.

Super-elastic characteristics may be simplistically described as follows. A metallic structure for example, a Nitinol tube that is in an Austenitic phase may be deformed to a particular shape or configuration by the application of mechanical energy. The application of mechanical energy causes a stress induced Martensitic phase transformation. In other words, the mechanical energy causes the Nitinol tube to transform from the Austenitic phase to the Martensitic phase. By utilizing the appropriate measuring instruments, it can be determined that the stress from the mechanical energy causes the temperature to drop in the Nitinol tube. Once the mechanical energy or stress is released, the Nitinol tube undergoes another mechanical phase transformation back to the Austenitic phase and thus its original or programmed shape. As described above, the original shape is programmed by well know techniques.

Medical devices constructed from Nitinol are typically utilized in both the Martensitic phase and/or the Austenitic phase. The Martensitic phase is the low temperature phase. A material is in the Martensitic phase is typically very soft and malleable. These properties make it easier to shape or configure the Nitinol into complicated or complex structures. The Austenitic phase is the high temperature phase. A material in the Austenitic phase is generally much stronger than the material in the Martensitic phase. Typically, many medical devices are cooled to the Martensitic phase for manipulation and loading into delivery system and heated again for deployment. For example, apparatus 100 assumes a first, contracted shape for placement into a sheath 134. When the apparatus 100 is deployed at body temperature, it returns to the Austenitic phase, or a second, expanded shape as shown in Figure 4.

Although constructed from a super elastic material, the frame 113 and membrane 130 expand axially at different rates during loading or compression of the apparatus 100 into the sheath 134. In order to maintain flexibility of the apparatus 100 as it is loaded into the catheter 134, it is preferred that the distal ends of the frame and membrane 130 are not connected. Instead, the membrane 130 is attached directly to the wire or tether 102 and disposed over the frame 113.

As shown in Figure 1, the separation edge 111 comprises at least two members 110a and 110b that are joined together. Alternatively, separation edge 111 may comprise a single hoop attached at its distal end to the wire or tether 102. The proximal end of the separation edge 111 is connected to the distal end of wire or tether 102. Each member 110a, 110b may also be attached directly to the wire at their proximal ends rather than to each other. Upon deployment from sheath 134, it is desired that the separation edge 111 maintain contact with the inside of the conduit forming a tight seal therewith. In one embodiment of the invention, the members 110a and 110b are arcuate to accommodate generally eccentric conduits. For example, the distance (D) between the peaks of the members 110a and 100b ranges from 1.00mm to 7.00mm. An opening 136 is defined between the members 110a and 110b serving as an inlet through which matter passes into the interior 115 after it is dislodged from the walls of a conduit Alternatively, each of the members 110a and 110b may have a different shape so as to conform the separation edge to the shape of the conduit 140.

As shown in Figures 1 and 4 Members 110a and 110b are joined together at their distal ends and at their proximal ends such that there is an acute separation angle 108 between the proximal ends of members 110a and 110b. In addition, separation edge 111 is attached to the wire 102 at an angle 109 providing an offset cutting surface. As the separation edge 111 is pulled toward matter to be removed from the wall of a conduit or vessel, the proximal end of separation edge 111, which is attached to the wire 102 and is more rigid, will be the first to contact the matter. The increased rigidity of the proximal end of separation edge 111 along with the acute separation angle 108 will apply shearing force to remove the matter from the wall of the conduit. As the separation edge is further articulated proximally toward the sheath 134, the slanted or offset distal portion of the separation edge 111 will urge the matter away from the wall of the conduit and into the interior 115 of the frame 113 through inlet 136.

The apparatus 100 is sized to allow for passage into small conduits such as blood vessels found within the vasculature of the brain. In addition, the apparatus 100 must also deploy to the proper configuration to ensure filtration or removal of matter 144 from within conduit 140. For example, apparatus 100 will have the ability to be compressed down to sizes of a microcatheter inner diameter of between 0.46 mm to 0.53 mm (0.018"-0.021") and still deploy or expand to sizes between 5.00 mm to 7.01mm (0.197"-0.276"). Due to the relatively small dimensions of apparatus 100, the separation edge 111 experiences high stress and strain due to the force required for removing matter that is entrapped within, or fixed to, the walls 142 of a conduit 140. Without proper support, the separation edge 111 would experience strain to the point where it assumes its first, contracted shape and fails to remove the matter from the conduit 140. Increasing the modulus of elasticity of the separation edge 111 is not an option since this would inhibit the deliverability and deployment of the apparatus 100 within small conduits. Thus, the separation edge 111 requires additional structure that will resist strain, but not inhibit deployment and deliverability.

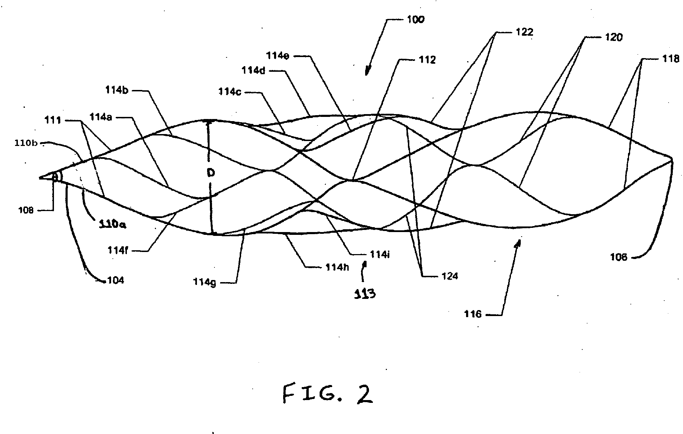

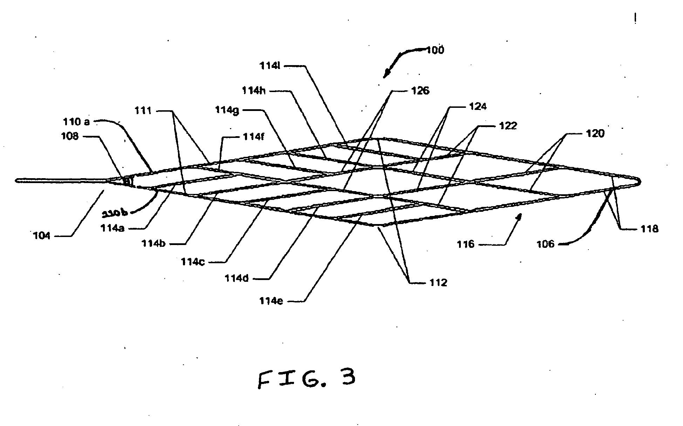

Frame 113 is linked to the separation edge 111 and provides support thereto preventing deformation as edge 111 engages matter 144 and removes it from the wall 142 of the conduit 140. One example of a frame 113 that provides the desired support is shown if Figures 2 and 3. The frame 113 comprises a plurality of struts that are attached to the separation members 110a and 110b.

A first group of struts 114 are connected to the separation edge 111 and to a second group of struts 116 that are joined at the distal end 106 of the frame 113. As seen in Figure 3, struts 114a-114e are evenly distributed along the length of member 110b. In particular, strut 114a is attached to the proximal end of member 110b, struts 114b and 114c are attached to the mid portion of member 110b, and struts 114d and 114e are attached to the distal portion of member 110b. Struts 114f and 114g are attached to the mid portion of member 110a and struts 114h and 114i are attached to the distal portion of member 110a. In order to optimize deployment of the apparatus 100, there are no struts attached to the proximal portion of member 110a. Although this is a preferred configuration, it may be necessary to provide additional support to separation edge 111 in which case additional struts 114 can be attached to member 110a or either of members 110a and 110b. First struts 114 are attached to strut the second set of struts 116, in particular, strut sets 122 and 126 described below.

The first group of struts 114 distributes forces from the members 11 10a and 110b to the distal end of frame 113 via the second group of struts located distally of the separation edge 111. The second group of struts 116 comprises an outer strut set 118 that spans from the distal end 106 of the frame 113 to the distal end of separation edge 111 and a plurality of inner strut sets 120, 122, 124 and 126 interspersed between the outer strut set 118 as described herein. The distal end of each strut of set 120 is connected to the distal portion of outer strut set 118 while the distal end of each strut of set 122 is connected to the proximal portion of outer strut set 118. The proximal ends of the struts of set 126 are connected together and the distal ends of each strut of set 126 are connected to the proximal end of strut sets 122 and 124. The distal ends of the struts of set 124 are connected together and to the proximal ends of the struts of set 120 that are also joined together.

Frame 113 forms a scaffold or space truss that distributes forces across the second struts 116. Although a particular form of truss is illustrated frame 113 may take on a variety of spatial configurations known in the art. In addition, the strut configuration within the frame 113 can be varied. Yet another option is to vary the thickness or width of the struts. Alternatively, the materials used to construct the frame 113 or separation edge 111 can be varied throughout the apparatus to achieve the necessary rigidity and flexibility of apparatus 100.

When the apparatus 100 is delivered to a targeted site in the conduit 140 via a sheath 134 or other delivery device it is contracted within the sheath 134 to a first diameter. Contracting the frame 113 and separation edge 111 to a small diameter increases stiffness thereby limiting the minimum delivery profile achievable. In addition, the apparatus 100 may deform when contracted jeopardizing optimal deployment. If the apparatus 100 fails to properly deploy the separation edge 111 will not assume the proper cutting angle and will fail to seal against the walls of the conduit potentially allowing matter to escape downstream. Of even greater concern is if the separation edge 111 deforms into a traumatic configuration causing damage to the walls of the conduit 140. In order to ensure proper deployment of the apparatus, at least one deployment section 112 is disposed along the separation edge 111. The deployment section 112 allows the apparatus 100 to assume its first contracted shape, without increasing stiffness, and allows for deployment in a predictable fashion. If desired, a plurality of deployment sections 112 can be distributed along the frame 113 and separation edge 111 depending upon the delivery profile.

In one embodiment of the invention, the deployment section 112 comprises a pre-formed point deformation such as a kink as shown in greater detail in Figure 1A. The prior art discusses kinks as being detrimental to loading and deployment of the apparatus 100 from a sheath 134. In contrast to the prior art, the present invention employs a kink specifically formed in an area that will assure that the apparatus 100 is folded within the sheath 134 and deployed in a predictable manner. As shown in figure 1A, the kink 112 is located between the point where outer strut set 118 and members 110a and 1 10b are joined together. The kink 112 has substantially the same, or greater, thickness (t) as members 110a and 110b and struts 118. Kink 112 is in the form of a pre-bend that will allow the apparatus 100 to compress within the sheath 134 without increasing stiffness and to deploy to the desired configuration. Moreover, kink 112 will allow for reloading of the apparatus within a sheath or delivery device 134

Alternatively, the deployment section 112 comprises a material interposed between frame 113 and separation edge 111 that is more flexible than the material that frame 113 and separation edge 111 is constructed form. In this instance the more flexible material allows predictable bending at the point where folding of the apparatus 100 occurs when it is contracted into catheter 134. In yet another embodiment, the modulus of elasticity of the frame 113 and the separation edge 111 may be varied such that bending occurs at deployment section 112 disposed there between.

When employed as a filter, the apparatus 100 is positioned distally of the matter 144 to be removed where it ensures that pieces of the matter 144 do not escape downstream as another device acts upon and removes the matter 144 from conduit 140. When employed to actively remove the matter 144, the apparatus 100 is first positioned distally of the matter 144 and is then pulled proximally whereby the apparatus engages the matter 144 and detaches it from the wall 142 of the conduit.

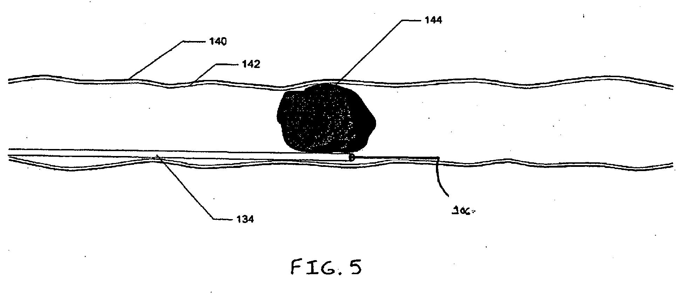

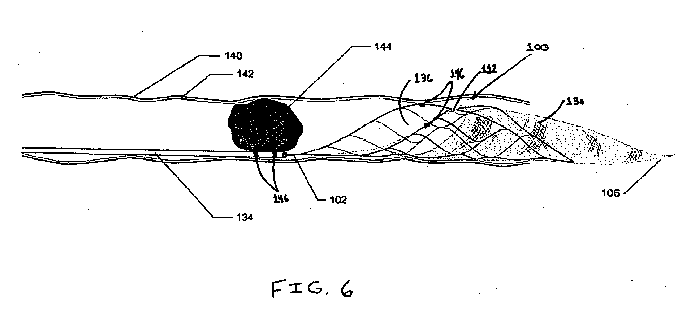

As shown in Figure 5, a sheath or catheter 134 is guided through conduit 140 to a position distal of the matter 144 to be removed. The matter 144 may be attached to the wall 142 of the conduit 140 and usually occludes substantially all of conduit 140. Thus, the apparatus 100 is compressed to a small diameter to allow for the use of a low profile sheath 134 to navigate around matter 144. As shown in Figures 5 and 6, when the sheath 134 is in position, the apparatus 100 is pushed from the sheath via actuation of the wire or tether 102. Alternatively, the wire 102 may be held in place to prevent movement and the sheath 134 may be withdrawn exposing the apparatus 100. The distal end of the apparatus 106 emerges from the sheath 134. The distal end 106 of the apparatus 100 includes atraumatic tip that will not puncture the walls 142 of the conduit. Thereafter, the super elastic frame 113 and separation edge 111 emerge from the sheath 134 resuming their remembered or deployed shape. A set of marker bands 146 on the sheath 134 and apparatus 100 allows for alignment of the apparatus 100 into a position to ensure optimal filtration or alignment of the separation edge 111 with the matter 144.

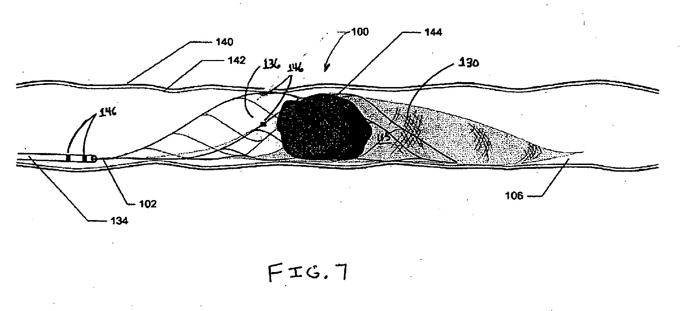

As shown in Figure 7, once the apparatus 100 has been properly aligned, it is pulled proximally, towards sheath 134. The separation edge 111 engages the matter 144 and slides, or cuts, it away from the wall 142 of the conduit 140. The matter 144 passes through inlet 136 and into the interior of the frame 115 where it is captured. Membrane 130 prevents pieces of matter 144 that may have become dislodged from escaping the interior 115. Fluid from the conduit 140 is free to pass through the interstices of membrane 130 ensuring that proper flow of fluid through conduit 140 is maintained. Thereafter, the sheath 134 and apparatus 100 are withdrawn proximally through the conduit 140 to a point where the matter 144 is removed.

## Claims

1. An apparatus comprising:
a separation edge (111) which is provided by at least two curved separation members (110a, 110b), each of the separation members having a distal end and a proximal end, the separation members being joined together at their distal ends and at their proximal ends, and defining an inlet between them, the angle between the separation members at their proximal ends being acute so as to form a sharp lead section at the proximal end of the separation edge, and the separation members being slanted relative to the longitudinal axis of the apparatus to provide a cutting surface which is angled,
a frame (113) having a proximal end at which the frame is attached to the separation edge and a distal end (106) and being constructed from a super-elastic material, the frame comprising a first group of struts (114) and a second group of struts (116), wherein the second group of struts (116) is located distally of the distal ends of the separation members and the first group of struts (114) distributes forces from the separation members to the distal end (106) of the frame (113) via the second group of struts, the second group of struts (116) including:
(i) first and second outer struts (118) which span from the distal ends of the separation members to the distal end of the frame and which are connected to one another only at the distal ends of the separation members and at the distal end of the frame, and
(ii) a plurality of inner strut sets (120, 122, 124, 126) which are interspersed between the first and second outer struts (118),
a deployment section (112) located along the separation edge at the intersection of the separation members (110a, 110b) and the first and second outer struts (118) and having substantially the same cross sectional area as the separation edge, the deployment section facilitating elastic contraction of the separation edge and the frame to a small diameter for placement in a delivery device, and
a membrane (130) attached to the frame.

2. The apparatus (100) of claim 1 in which a proximal end of the separation edge (111) is attached to a wire (102) such that the separation edge (111) and frame (113) may be pushed from or pulled into a sheath (134) causing the edge (111) and frame (113) to expand or contract.

3. The apparatus of claim 1 in which the deployment section comprises:
(i) a preformed kink (112),
(ii) a material interposed between the frame (113) and the separation edge (111) that is more flexible than the material that the frame (113) and the separation edge (111) is constructed from, or
(iii) a portion in which the modulus of elasticity of the frame (113) and the separation edge is varied such that bending occurs at the deployment section (112) disposed between them.

4. The apparatus (100) of claim 1 in which the frame (113) includes a plurality of marker bands that are visible when inserted into a human body.

5. The apparatus (100) of claim 1 in which the distal end of the frame (113) comprises an atraumatic tip.

6. The apparatus (100) of claim 2 in which the membrane (130) is attached to the wire (102) and is disposed over the frame (113) such that an opening in the membrane (130) corresponds to the inlet.

7. The apparatus (100) of claim 1 in which the membrane (130) comprises a net constructed from a thin film metal deposited on a frame.

8. The apparatus (100) of claim 1 in which the membrane (130) comprises a braided tube constructed from wire.

9. The apparatus (100) of claim 1 in which the frame (113), the membrane (130), separation edge (111) and deployment section (112) are constructed from a superelastic material.

## Patentansprüche

1. Vorrichtung, welche Folgendes umfasst:
einen Trennrand (111), welcher von zumindest zwei gekrümmten Trennelementen (110a, 110b) bereit gestellt ist, wobei jedes der Trennelemente ein distales Ende und ein proximales Ende aufweist, wobei die Trennelemente an ihren distalen Enden und an ihren proximalen Enden zusammengeführt sind und einen Einlass zwischen sich definieren, wobei der Winkel zwischen den Trennelementen an ihren proximalen Enden spitz ist, so dass ein scharfer Führungsabschnitt an dem proximalen Ende des Trennrandes gebildet ist, und wobei die Trennelemente relativ zu der Längsachse der Vorrichtung geneigt sind, um eine Schneidoberfläche bereit zu stellen, welche angewinkelt ist,
einen Rahmen (113), welcher ein proximales Ende, an welchem der Rahmen an dem Trennrand angebracht ist, und ein distales Ende (106) aufweist, und welcher aus einem überelastischen Material erstellt ist, wobei der Rahmen eine erste Gruppe von Streben (114) und eine zweite Gruppe von Streben (116) umfasst, wobei sich die zweite Gruppe von Streben (116) distal von den distalen Enden der Trennelemente befindet, und wobei die erste Gruppe von Streben (114) Kräfte von den Trennelementen über die zweite Gruppe von Streben an das distale Ende (106) des Rahmens (113) verteilt,
wobei die zweite Gruppe von Streben (116) Folgendes aufweist:
(i) eine erste und eine zweite äußere Strebe (118), welche von den distalen Enden der Trennelemente zu dem distalen Ende des Rahmens führen und welche miteinander nur an den distalen Enden der Trennelemente und an dem distalen Ende des Rahmens verbunden sind, und
(ii) mehrere Mengen (120, 122, 124, 126) innerer Streben, welche zwischen der ersten und der zweiten äußeren Strebe (118) verteilt sind,
einen Entfaltungsabschnitt (112), welcher sich entlang des Trennrandes an dem Schnittpunkt der Trennelemente (110a, 110b) und der ersten und der zweiten äußeren Strebe (118) befindet und im Wesentlichen die gleiche Querschnittsfläche wie der Trennrand aufweist, wobei der Entfaltungsabschnitt ein elastisches Zusammenziehen des Trennrandes und des Rahmens auf einen kleinen Durchmesser zur Platzierung in einer Zuführungsvorrichtung erleichtert, und
eine an dem Rahmen angebrachte Membran (130).

2. Vorrichtung (100) nach Anspruch 1, wobei ein proximales Ende des Trennrandes (111) an einem Draht (102) angebracht ist, so dass der Trennrand (111) und der Rahmen (113) aus einer Hülle (134) geschoben oder in eine Hülle (134) gezogen werden können, wodurch bewirkt wird, dass sich der Rand (111) und der Rahmen (113) ausdehnen oder zusammenziehen.

3. Vorrichtung nach Anspruch 1, wobei der Entfaltungsabschnitt Folgendes umfasst:
(i) einen vorgeformten Knick (112),
(ii) ein Material, welches zwischen dem Rahmen (113) und dem Trennrand (111) angeordnet ist und welches flexibler ist als das Material, aus welchem der Rahmen (113) und der Trennrand (111) erstellt sind, oder
(iii) einen Bereich, in welchem das Elastizitätsmodul des Rahmens (113) und des Trennrandes variiert ist, so dass ein Biegen bei dem zwischen ihnen angeordneten Entfaltungsabschnitt (112) auftritt.

4. Vorrichtung (100) nach Anspruch 1, wobei der Rahmen (113) mehrere Markierungsbänder aufweist, welche sichtbar sind, wenn sie in einen menschlichen Körper eingerührt sind.

5. Vorrichtung (100) nach Anspruch 1, wobei das distale Ende des Rahmens (113) eine atraumatische Spitze umfasst.

6. Vorrichtung (100) nach Anspruch 2, wobei die Membran (130) an dem Draht (102) angebracht ist und über dem Rahmen (113) angeordnet ist, so dass eine Öffnung in der Membran (130) dem Einlass entspricht.

7. Vorrichtung (100) nach Anspruch 1, wobei die Membran (130) ein Netz umfasst, welches aus einem auf einem Rahmen abgeschiedenen Dünnschichtmetall erstellt ist.

8. Vorrichtung (100) nach Anspruch 1, wobei die Membran (130) eine aus einem Draht erstellte Flechthülse umfasst.

9. Vorrichtung (100) nach Anspruch 1, wobei der Rahmen (113), die Membran (130), der Trennrand (111) und der Entfaltungsabschnitt (112) aus einem überelastischen Material erstellt sind.

## Revendications

1. Appareil comprenant :
un bord de séparation (111) qui est fourni par au moins deux éléments de séparation incurvés (110a, 110b), chacun des éléments de séparation ayant une extrémité distale et une extrémité proximale, les éléments de séparation étant assemblés au niveau de leurs extrémités distales et au niveau de leurs extrémités proximales, et définissant une entrée entre eux, l'angle entre les éléments de séparation au niveau de leurs extrémités proximales étant aigu afin de former une section d'attaque pointue au niveau de l'extrémité proximale du bord de séparation, et les éléments de séparation étant inclinés par rapport à l'axe longitudinal de l'appareil afin de fournir une surface de coupe qui est angulaire,
un châssis (113) ayant une extrémité proximale à laquelle le châssis est fixé au bord de séparation et une extrémité distale (106) et étant construit à partir d'un matériau super élastique, le châssis comprenant un premier groupe de montants (114) et un second groupe de montants (116), dans lequel le second groupe de montants (116) est positionné à distance des extrémités distales des éléments de séparation et le premier groupe de montants (114) répartit les forces des éléments de séparation à l'extrémité distale (106) du châssis (113) via le second groupe de montants, le second groupe de montants (116) comprenant :
(i) des premiers et seconds montants (118) qui s'étendent des extrémités distales des éléments de séparation à l'extrémité distale du châssis et qui sont raccordées les uns aux autres uniquement aux extrémités distales des éléments de séparation et à l'extrémité distale du châssis, et
(ii) une pluralité d'ensembles de montants internes (120, 122, 124, 126) qui sont parsemés entre les premiers et seconds montants externes (118),
une section de déploiement (112) positionnée le long du bord de séparation à l'intersection des éléments de séparation (110a, 110b) et des premiers et seconds montants externes (118) et ayant sensiblement la même surface transversale que le bord de séparation, la section de déploiement facilitant la contraction élastique du bord de séparation et du châssis à un petit diamètre pour la mise en place dans un dispositif de pose, et
une membrane (130) fixée au châssis.

2. Appareil (100) selon la revendication 1, dans lequel une extrémité proximale du bord de séparation (111) est fixée à un fil (102) de sorte que le bord de séparation (111) et le châssis (113) peuvent être poussés ou tirés dans une gaine (134) pour amener le bord (111) et le châssis (113) à s'expanser ou à se contracter.

3. Appareil selon la revendication 1, dans lequel la section de déploiement comprend :
(i) un noeud préformé (112),
(ii) un matériau intercalé entre le châssis (113) et le bord de séparation (111) qui est plus flexible que le matériau à partir duquel le châssis (113) et le bord de séparation (111) sont construits, ou bien
(iii) une partie dans lequel le module d'élasticité du châssis (113) et le bord de séparation est modifié de sorte que la flexion se produit au niveau de la section de déploiement (112) disposée entre eux.

4. Appareil (100) selon la revendication 1, dans lequel le châssis (113) comprend une pluralité de bandes de marqueur qui sont visibles lorsqu'elles sont insérées dans un corps humain.

5. Appareil (100) selon la revendication 1, dans lequel l'extrémité distale du châssis (113) comprend une pointe atraumatique.

6. Appareil (100) selon la revendication 2, dans lequel la membrane (130) est fixée au fil (102) et disposée sur le châssis (113) de sorte qu'une ouverture dans la membrane (130) correspond à l'entrée.

7. Appareil (100) selon la revendication 1, dans lequel la membrane (130) comprend un filet construit à partir d'un métal en film fin disposé sur un châssis.

8. Appareil (100) selon la revendication 1, dans lequel la membrane (130) comprend un tube tressé construit à partir du fil.

9. Appareil (100) selon la revendication 1, dans lequel le châssis (113), la membrane (130), le bord de séparation (111) et la section de déploiement (112) sont construits à partir d'un matériau super élastique.
